# EUROPEAN PATENT SPECIFICATION

(11) **EP 3 668 999 B1**
(45) Date of publication and mention of the grant of the patent: **26.07.2023**
(21) Application number: 18773818.2
(22) Date of filing: 20.08.2018
(51) Int. Cl.: C12Q 1/6844, C12Q 1/6818

(54) **METHODS AND KITS FOR DETECTION OF NUCLEIC ACID MOLECULES**
VERFAHREN UND KITS ZUR DETEKTION VON NUKLEINSÄUREMOLEKÜLEN
PROCÉDÉS ET TROUSSES DE DÉTECTION DE MOLÉCULES D'ACIDE NUCLÉIQUE

(30) Priority: 18.08.2017 GB 201713251
(43) Date of publication of application: 24.06.2020
(73) Proprietor: LGC Genomics Limited, Middlesex TW11 0LY (GB)
(72) Inventor: HOWARD, Rebecca Louise, Teddington TW11 0LY (GB)
(74) Representative: Potter Clarkson
(86) International application number: PCT/GB2018/052360
(87) International publication number: WO 2019/034898

(56) References cited:
- WO-A1-2014/135872
- WO-A1-2017/108663
- US-A1- 2013 171 643
- US-A1- 2013 323 738
- US-A1- 2016 076 083
- GAOLIAN XU ET AL: "Cross Priming Amplification: Mechanism and Optimization for Isothermal DNA Amplification", SCIENTIFIC REPORTS, vol. 2, 3 February 2012 (2012-02-03), XP055121152, DOI: 10.1038/srep00246 cited in the application
- Y. KIMURA ET AL: "Optimization of turn-back primers in isothermal amplification", NUCLEIC ACIDS RESEARCH, vol. 39, no. 9, 1 May 2011 (2011-05-01), pages e59-e59, XP055164865, ISSN: 0305-1048, DOI: 10.1093/nar/gkr041

## Description

### FIELD OF THE INVENTION

The present invention relates to methods and kits for detection of nucleic acid molecules, particularly polynucleotide amplification products formed in an isothermal polynucleotide amplification reaction.

### BACKGROUND OF THE INVENTION

Sequence-specific isothermal nucleic acid amplification techniques offer advantages over traditional polymerase chain reaction (PCR), because they can provide rapid and sensitive molecular detection without the need for thermal cycling equipment as used in PCR. They may find particular utility in field or point-of-care molecular diagnostics due to their suitability for use with lower-powered and portable instrumentation.

In contrast to PCR, which denatures double-stranded DNA (dsDNA) with heat, isothermal amplification methods typically use enzymatic activity to separate the strands of a target polynucleotide, and permit the hybridisation of sequence specific oligonucleotide primers. The amplification reaction is driven by multiple rounds of primer hybridisation, primer extension and strand separation, and is performed at a single temperature. Although many amplification systems are currently in use, not all are simple enough to provide specific detection in a short time frame. Those that are able to lend themselves to rapid amplification typically use a strand-displacement polymerase which serves the dual function of strand separation and primer extension. Such methods include loop-mediated isothermal amplification (LAMP) (Notomi et al, 2000, Nucleic Acids Res.28(12):E63, Loop-mediated isothermal amplification of DNA; Nagamine et al, 2002, Mol Cell Probes. 16(3):223-9, Accelerated reaction by loop-mediated isothermal amplification using loop primers), Smart Amplification (SMAP) (Lezhava and Hayashizaki, 2009, Methods Mol Biol. 578:437-51 Detection of SNP by the isothermal smart amplification method, and Cross-Priming Amplification (CPA) (Xu et al, 2012, Sci Rep. 2:246, Cross priming amplification: mechanism and optimization for isothermal DNA amplification). Each of these is able to produce abundant polynucleotide amplification products from a target polynucleotide using a strand-displacement polymerase in conjunction with complex oligonucleotide primers.

While many earlier isothermal amplification methods have relied on the detection of target nucleic acids using turbidity or intercalating dye methods, in recent years detection using fluorescent probes has become more common. Typical fluorescent probe detection methods involve the use of probes which are designed to bind to the amplified target polynucleotide in a sequence-specific manner. For example, TaqMan probes are dual-labeled oligonucleotides which hybridise to target polynucleotides. A 5'-3' exonuclease activity of a DNA polymerase displaces and degrades the probe, releasing the fluorophore from the quencher. Such probes have successfully been used in multiplex isothermal amplification reactions in which a helicase is included to separate the strands of dsDNA target (Tong et al, 2008, Biotechniques. 45(5):543-57. doi: 10.2144/000112959, Development of isothermal TaqMan assays for detection of biothreat organisms). HyBeacon probes are single-stranded oligonucleotides with one or more internal bases labelled with a fluorescent dye. They are more fluorescent when hybridised to a complementary target than when single stranded, and their hybridisation to a target can be monitored using melting or annealing curve analysis. HyBeacon probes have been successfully used to detect amplification products of isothermal amplification reactions, and sequence variations in the target sequence can be detected as different melting peaks (Howard etal, 2015, Mol Cell Probes. 29(2):92-8, doi: 10.10161j.mcp.2014.12.001, Rapid detection of diagnostic targets using isothermal amplification and HyBeacon probes--a homogenous system for sequence-specific detection). For example, a HyBeacon probe can be used to distinguish different alleles in an amplification product, by virtue of being fully complementary to one allele, and incorporating a mismatch with respect to another allele.

The LAMP technique has recently been adapted for the detection of multiple targets, by adaptation of one of the target-specific LAMP primers for each amplification reaction to contain a quencher or fluorophore, and the provision of a complementary oligonucleotide containing a fluorophore or quencher, as appropriate, which forms a duplex with the LAMP primer (Tanner et al, 2012, Biotechniques. 53(2):81-9. doi: 10.2144/0000113902, Simultaneous multiple target detection in real-time loop-mediated isothermal amplification). Strand separation of the duplex region during the amplification reaction results in a gain of fluorescence signal. The use of different fluorophore/quencher pairs permits detection of multiple targets. However, the selection of targets depends on the specificity of the complementary labelled oligonucleotide for the labelled target-specific LAMP primer, and a lack of cross-reactivity with other target-specific LAMP primers used in a multiplex reaction. In practice, the method has been used to detect highly divergent target sequences derived from human, *E*. *coli, C. elegans* and bacteriophage DNA in multiplex.

There is a need for detection methods which do not rely solely on differences between the nucleotide sequence of target sequences to enable different amplification products in isothermal amplification reactions to be distinguished. The present invention provides a method in which a detection cassette is incorporated into the amplification product, and the presence of the cassette or its complement is detected by virtue of hybridisation to a cassette oligonucleotide, which is typically labelled. Different cassettes or their complements can be detected with differently labelled cassette oligonucleotides, permitting even closely related products to be distinguished in multiplex reactions. Suitable cassette oligonucleotides have been developed for use in conventional PCR (US 2007/117108 A1, US 2013/252238 A1 and WO 2014/135872 A1) but have not previously been utilised in isothermal amplification reactions, which require complex multi-domain oligonucleotide primers.

The listing or discussion of a prior-published document in this specification should not be taken as an acknowledgement that the document is part of the state of the art or is common general knowledge.

### SUMMARY OF THE INVENTION

The present invention is defined by the appended claims.

According to the invention there is provided a method for the detection of one or more polynucleotide amplification products, the method comprising the steps of:
a) providing one or more oligonucleotide primer groups, each group comprising one or more oligonucleotide primer sets, each set characterised by:
   i) a first oligonucleotide primer (forward primer) comprising a fluorescence cassette-specific portion and a 3' downstream target-specific portion; and
   ii) one or more target-specific oligonucleotide primers;

   wherein the oligonucleotide primers in a particular set are each suitable for hybridisation to a strand of a target polynucleotide to permit formation of an amplification product of the target polynucleotide in an isothermal polynucleotide amplification reaction;
   and wherein the first oligonucleotide primer of each set in the same group contains a fluorescence cassette-specific portion that is capable of hybridising to the complement of the fluorescence cassette-specific portion of the first oligonucleotide primer of any set in the same group;
b) providing one or more cassette oligonucleotide sets, each set characterised by:
   i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
   ii) a second cassette oligonucleotide labelled with an acceptor moiety (for example a quencher moiety);
   wherein the cassette oligonucleotides of each set hybridise to one another to form a fluorescent quenched pair;
c) initiating an isothermal polynucleotide amplification reaction in the presence of a strand displacement DNA polymerase thereby generating (if the target polynucleotide is present) a complementary sequence to at least a portion of the relevant first oligonucleotide primer, such that the relevant second (acceptor, for example quencher, labelled) cassette oligonucleotide is less able to hybridise to the relevant first (fluorescently labelled) cassette oligonucleotide, whereby a signal is generated; and
d) detecting the signal that is generated.

Kits suitable for use in such a method are also provided.

### BRIEF DESCRIPTION OF THE DRAWINGS

Figure 1A: Target sequences utilised in loop-mediated isothermal amplification (LAMP) are shown together with the corresponding classical LAMP primers. In an exemplary method of the invention, a cassette universal sequence is incorporated into one of the LAMP 'inner primers' between the F1c and F2 (or B1c and B2) sequences as shown. This incorporates the universal cassette sequence into one of the LAMP 'loops' where it is accessible.
Figure 1B: This figure demonstrates, based on the structures defined in Figure 1A, the mechanism by which the universal cassette sequence can be incorporated into a LAMP reaction product, replicated and its complement detected. This figure shows only the relevant part of the full LAMP reaction.
Figure 2: This figure shows both real-time (A/B) and end-point (C) data generated when combining the SNP genotyping LAMP reaction described in Example 1 with the universal cassette isothermal detection system. The figure shows:
   A) Real-time data generated in the FAM detection channel of a Roche LightCycler 480 instrument. The fluorescence traces pertaining to multiple reactions are overlaid, with one group of traces showing development of fluorescence over time, and the other group showing minimal or no fluorescence development over time. A flat trace is for a no target control.
   B) Real-time data generated in the HEX detection channel of a Roche LightCycler 480 instrument. The fluorescence traces pertaining to multiple reactions are overlaid, with one group of traces showing development of fluorescence over time, and the other group showing minimal or no fluorescence development over time. The members of the high and low fluorescence groups correspond respectively to the members of the low and high fluorescence groups shown in A. A flat trace is for a no target control.
   C) An end-point data plot produced post-amplification, in which the Y axis shows fluorescence intensity of the HEX channel and the X axis shows fluorescence intensity of the FAM channel. The two genotyping clusters are clearly distinguished.

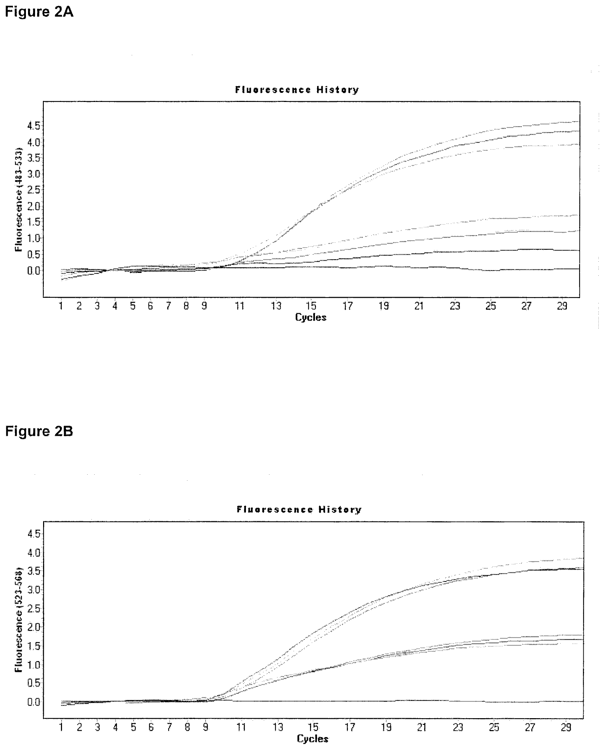

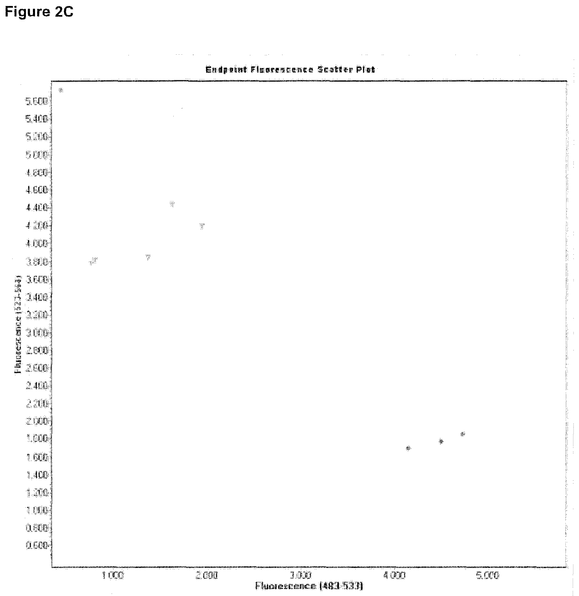

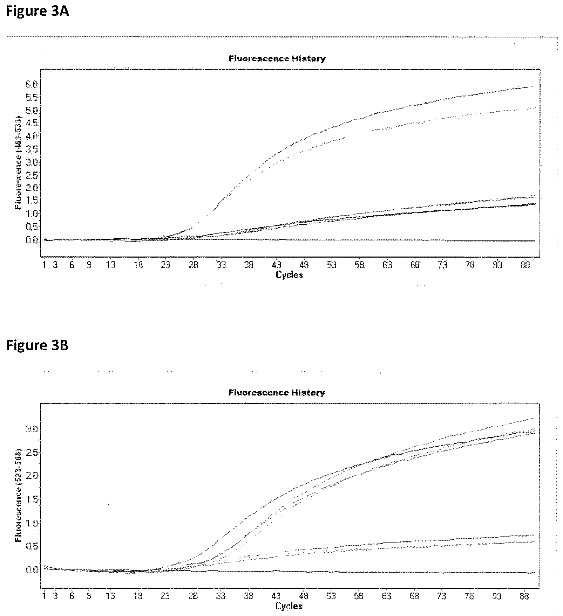

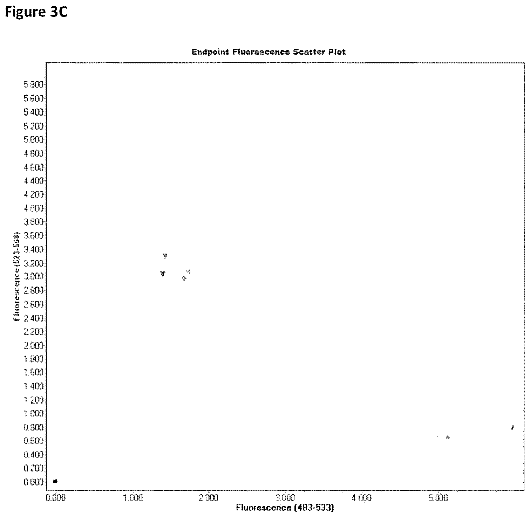

Figure 3: This figure shows both real-time (A/B) and end-point (C) data generated in Example 2, and as described for Figure 2.

### DETAILED DESCRIPTION OF PREFERRED EMBODIMENTS OF THE INVENTION

A first aspect of the invention provides a method for the detection of one or more polynucleotide amplification products, the method comprising the steps of:
a) providing one or more oligonucleotide primer groups, each group comprising one or more oligonucleotide primer sets, each set characterised by:
   i) a first oligonucleotide primer (forward primer) comprising a fluorescence cassette-specific portion and a 3' downstream target-specific portion; and
   ii) one or more target-specific oligonucleotide primers;

   wherein the oligonucleotide primers in a particular set are each suitable for hybridisation to a strand of a target polynucleotide to permit formation of an amplification product of the target polynucleotide in an isothermal polynucleotide amplification reaction;
   and wherein the first oligonucleotide primer of each set in the same group contains a fluorescence cassette-specific portion that is capable of hybridising to the complement of the fluorescence cassette-specific portion of the first oligonucleotide primer of any set in the same group;
b) providing one or more cassette oligonucleotide sets, each set characterised by:
   i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
   ii) a second cassette oligonucleotide labelled with an acceptor moiety (for example a quencher moiety);
   wherein the cassette oligonucleotides of each set hybridise to one another to form a fluorescent quenched pair;
c) initiating an isothermal polynucleotide amplification reaction in the presence of a strand displacement DNA polymerase thereby generating (if the target polynucleotide is present) a complementary sequence to at least a portion of the relevant first oligonucleotide primer, such that the relevant second (acceptor, for example quencher, labelled) cassette oligonucleotide is less able to hybridise to the relevant first (fluorescently labelled) cassette oligonucleotide, whereby a signal is generated; and
d) detecting the signal that is generated.

Step (a) above provides one or more oligonucleotide primer groups, each group comprising one or more oligonucleotide primer sets which are suitable for generating an amplification product of a target polynucleotide in an isothermal polynucleotide amplification reaction. The first oligonucleotide primer in each set, also known as the "tailed primer", comprises both a 3' downstream target-specific sequence and a fluorescence cassette-specific portion. The purpose of the fluorescence cassette-specific portion is to become incorporated into the amplification product, such that the complement of the fluorescence cassette-specific portion becomes available for hybridisation to a first cassette oligonucleotide labelled with a fluorescent moiety. In each set, the complement of the fluorescence cassette-specific portion of the first oligonucleotide primer is capable of hybridising to the first cassette oligonucleotide labelled with a fluorescent moiety. Typically, the fluorescence cassette-specific portions of the first oligonucleotide primer in each set are identical. Typically, they are at least in part identical to the sequence of the first cassette oligonucleotide. In the use of the method, the first cassette oligonucleotide becomes hybridised to the or each amplification product of the or each oligonucleotide primer set, generating a signal. Suitably, the method involves more than one oligonucleotide primer group. Suitably, each oligonucleotide primer group incorporates a different fluorescence cassette-specific portion into the amplification product, which becomes hybridised to a different first cassette oligonucleotide labelled with a different fluorescent moiety. In this way, multiple targets may be distinguished by virtue of generating differing fluorescence signals.

In step (b), each first cassette oligonucleotide labelled with a fluorescent moiety is provided together with a second cassette oligonucleotide labelled with an acceptor moiety, and the two hybridise to one another to form a fluorescent quenched pair. The first cassette oligonucleotide of the pair is capable of hybridisation to the complement of the fluorescence cassette-specific portion of the first oligonucleotide primer of any set in a given oligonucleotide primer group. In other words, no matter how many oligonucleotide primer sets there are in a given group, only one fluorescent quenched pair is needed per group. Suitably, the method involves more than one oligonucleotide primer group, and a different fluorescent quenched pair is provided for each group, generating a different fluorescence signal for each group in the operation of the method. In one embodiment, each set is designed to generate an amplification product from a single target, and each amplification product is detected by virtue of acquisition of a different fluorescence signal.

In step (c), an isothermal polynucleotide amplification reaction generates (if the target polynucleotide is present) a complementary sequence to at least a portion of, and typically all of the relevant first oligonucleotide primer. The first (fluorescently labelled) cassette oligonucleotide preferentially hybridises to the complement of the fluorescence cassette-specific portion in the amplification product, and thus the quenching of its fluorescence signal by the second cassette oligonucleotide is relieved. In the first round of the polynucleotide amplification reaction, a primer extension product is formed from the first oligonucleotide primer. The amplification of this product from a reverse primer then typically forms a complementary sequence to the relevant first oligonucleotide primer. However, it cannot be ruled out that this reverse primed amplification might terminate before the formation of a complementary sequence to the whole of the relevant first oligonucleotide primer is complete. The portion of the complementary sequence to the relevant first oligonucleotide primer must at least comprise at least a portion of, typically all of, the complement of the fluorescence cassette specific portion in order for the relevant second (acceptor, for example quencher, labelled) cassette oligonucleotide to be less able to hybridise to the relevant first (fluorescently labelled) cassette oligonucleotide (considered to be because the formed complementary sequence to the relevant first oligonucleotide primer hybridises with the first cassette oligonucleotide instead).

### Reaction conditions and components

A "polynucleotide amplification reaction" as in the method of the invention involves hybridisation of a nucleic acid primer molecule to a strand of a template polynucleotide molecule, and a template-dependent primer extension reaction, in which a strand displacement DNA polymerase extends the hybridised primer by adding a sequence of bases to the 3' terminus of the primer, which sequence is determined by the sequence of bases in the template strand. The formation of a target-specific primer extension product, resulting from the template-dependent primer extension reaction, can be considered to be the first round of a polynucleotide amplification reaction. Further rounds require strand separation of the primer extension product such that further primers can hybridise to one or other strand and undergo a primer extension reaction. In an isothermal polynucleotide amplification reaction, as opposed to a conventional polymerase chain reaction (PCR), strand separation following the formation of the target-specific primer extension product in the first round of the reaction is not effected by heating alone. In particular, it is not necessary to heat the reaction to a temperature above the temperature selected for primer extension. The method of the invention is carried out in the presence of a strand displacement DNA polymerase, thus permitting multiple rounds of the polynucleotide amplification reaction to be performed at a relatively constant temperature. Typically, the polynucleotide amplification reaction (and optionally also detection of the signal) is performed throughout at a temperature which is suitable for primer extension from the strand displacement DNA polymerase. In certain embodiments, the reaction may be periodically cooled to facilitate detection of the signal in step (d). The reaction also may be cooled at its end point, if appropriate, to facilitate detection of the signal.

The term Ta will be well known to those skilled in the art and refers to the temperature (typically set or programmed into the apparatus controlling the reaction parameters) at which significant primer extension occurs during a primer extension reaction. Typically the same Ta will be used substantially throughout a polynucleotide amplification reaction. Sometimes a different Ta (typically higher) will be used in initial rounds of a polynucleotide amplification reaction. Typically, Ta will not vary by more than 10°C, typically by not more than 5 °C, or not more than 2 °C from the point at which the reaction components are combined or the first primer extension reaction is performed, until the termination or completion of the reaction. A suitable Ta is suitable for the operation of the strand displacement DNA polymerase and is one which facilitates template-specific primer hybridisation and extension. A suitable reaction temperature may be between 50 °C and 75 °C, such as between 60 °C and 65 °C. An initial denaturation step may be performed prior to the first primer extension reaction to separate the strands of the target nucleic acid molecule. For example, the reaction mixture, with the exception of the strand displacement DNA polymerase, may be heated to about 95 °C for about 5 minutes, then chilled to about 4 °C, prior to the addition of the strand displacement DNA polymerase and incubation at the reaction temperature. An initial denaturation step may not be necessary. For example, Xu *et al,* 2012, *supra,* describe an isothermal method in which betaine was included in the reaction buffer to cause spontaneous formation of local denaturation bubbles at the reaction temperature.

*Bst* DNA polymerase, large fragment is a suitable strand displacement DNA polymerase and has an activity at elevated temperatures of, and is stable at 50 °C to 70 °C for >30 minutes. Variants of existing polymerases can be readily screened by performing a standard isothermal polynucleotide amplification reaction, such as a standard LAMP using lambda DNA (Nagamine, et al., Mol. Cel. Probes, 16:223-9 (2002), in which sufficient amplification of 5 ng lambda phage genomic DNA is considered to be threshold detection in less than 30 minutes at 60° C to 68° C using standard primer concentrations. Suitable polymerase variants include *Bst* 2.0 or *Bst* 2.0 WarmStart^{™} DNA polymerases or 9° N^{™} polymerase (New England Biolabs, Ipswich, Mass.), compared with wild-type *Bst* DNA polymerase, large fragment where the multiplex reaction time could be reduced to as little as 5-30 minutes without significant reduction in signal. Another suitable polymerase variant is *Bst* 3.0 (New England Biolabs, Ipswich, Mass.) which is an *in silico* designed homologue of *Bacillus stearothermophilus* DNA Polymerase I, Large Fragment engineered for improved isothermal amplification performance and increased reverse transcription activity. *Bst* 3.0 DNA Polymerase contains 5'→3' DNA polymerase activity with either DNA or RNA templates and strong strand displacement activity, but lacks 5'→3' and 3'→5' exonuclease activity.

Typically, the strand displacement DNA polymerase lacks 5'-3' exonuclease and/or 3'-5' exonuclease activity. A lack of 3'-5' exonuclease activity may promote amplification specificity. Typically, 5'-3' exonuclease activity is absent or at low levels to avoid or reduce degradation of the displaced strand. In certain embodiments, the strand displacement DNA polymerase is the only enzyme present in the reaction. In other embodiments, a further enzyme may be included, such as a mismatch-binding protein, for example *Thermus aquaticus* MutS, which may reduce mismatch and background amplification, as described in Lezhava and Hayashizaki, 2009, *supra,* in relation to the SMAP method. A 5'-3' exonuclease, if present, may be GspSSD DNA Polymerase I, Large fragment; GspSSD2.0 DNA Polymerase I, Large fragment; or GspM3.0 DNA Polymerase I, Large Fragment, all of which are thermostable and also have reverse transcriptase activity. An inorganic pyrophosphatase may be included to prevent accumulation of pyrophosphate during the reaction, for example the inorganic pyrophosphatase available from OptiGene Ltd, UK (Cat# ISO-001nd), or the thermostable pyrophosphatase of *Thermococcus litoralis* disclosed in WO 9612798. In an embodiment, the only enzymes present in the reaction are a strand displacement DNA polymerase and an inorganic pyrophosphatase.

In practicing the methods of the invention, it is necessary to produce a polynucleotide amplification mixture, e.g. a composition that includes all of the elements necessary for a polynucleotide amplification reaction to occur.

The reaction mixture includes the primers employed in the primer extension reaction. One or more oligonucleotide primer groups may be used, each group comprising one or more oligonucleotide primer sets. Each set may typically have a forwards and a reverse primer. As noted above, typically 1, 2, 3, 4 or more primer groups may be used. Each group may typically include one primer set (unless, for example, there is a reason why it is wished to measure the combined amplification products arising from two separate sets of primers). Each polynucleotide amplification mixture typically will comprise at least two or three forward primers. A corresponding reverse primer for each forward primer may also be present, but these may not be different, for example in a SNP genotyping reaction, where a common reverse primer may be used with several different forward primers.

The polynucleotide amplification mixture includes at least one pair of labelled oligonucleotides (the cassette oligonucleotide set or sets) which oligonucleotides hybridise to one another to form a fluorescent quenched pair, wherein one oligonucleotide is labelled with a fluorescent moiety and the other oligonucleotide is labelled with a quenching moiety. The fluorescent labelled oligonucleotide of each set comprises a sequence that is capable of hybridisation to the complement(s) of the fluorescence cassette-specific region of the first oligonucleotide primer (forward primer) of each primer set of a particular group.

The nucleic acid that serves as template may be single stranded or double stranded, where the nucleic acid is typically deoxyribonucleic acid (DNA), but may alternatively by ribonucleic acid (RNA). The length of the template nucleic acid may be as short as 20 bp, but usually be at least about 50 or 100 bp long, and more usually at least about 150 bp long, and may be as long as 1,000 or 10,000 bp or longer, e.g. 50,000 bp in length or longer, including a genomic DNA extract, digest thereof or crude lysate, etc. The nucleic acid may be free in solution, flanked at one or both ends with non-template nucleic acid, present in a vector, e.g. plasmid and the like, with the only criteria being that the nucleic acid be available for participation in the primer extension reaction. The template nucleic acid may be present in purified form, or in a complex mixture with other non-template nucleic acids, e.g. in cellular DNA preparation, etc.

The template nucleic acid may be derived from a variety of different sources, depending on the application for which the PCR is being performed, where such sources include organisms that comprise nucleic acids, i.e. viruses; prokaryotes, e.g. bacteria, archaea and cyanobacteria; and eukaryotes, e.g. vertebrates, including reptiles, fishes, birds, snakes, and mammals, e.g. rodents, primates, including humans. The template nucleic acid may be used directly from its naturally occurring source, e.g. as a crude lysate and/or it may preprocessed in a number of different ways, as is known in the art. In some embodiments, the template nucleic acid may be from a synthetic source.

The reaction mixture may contain various buffer components, salts, dNTPs, buffering agents, divalent cations, e.g. derived from magnesium salts, as known in the art. MgSO₄ may be included, typically within a range of 3.5mM - 5.5mM, such as at a concentration of 4 mM.

### Oligonucleotide primers

As noted above, each oligonucleotide primer set includes a first oligonucleotide primer (forward primer) comprising a fluorescence cassette-specific portion and a 3' downstream target-specific portion; and one or more target-specific oligonucleotide primers. The first oligonucleotide primer may include one or more further primer portions and likewise one or more of the target-specific oligonucleotide primers may comprise more than one primer portion. A "primer portion" is intended to mean a portion of a primer having a role in the formation of the polynucleotide amplification product. Typical primer portions are target-specific portions, but may also include self-annealing portions.

Suitably, the or each primer portion is designed to have a melting temperature (Tm) intended to be approximately 5 °C above the Ta of the reaction. The Tm of an oligonucleotide is the temperature in °C at which 50% of the molecules in a population of a single-stranded oligonucleotide are hybridised to their complementary sequence and 50% of the molecules in the population are not-hybridised to said complementary sequence. A commonly used formula for determining the Tm of a sequence is Tm=4(G+C)+2(A+T). The Tm of an oligonucleotide pair may also be measured empirically, for example Tm may be measured using melting curve analysis, e.g. using a Roche LightCycler 480 instrument on a 96-well white plate. The Tm may be tested in standard reaction buffer in the absence of polymerase. Standard reaction buffer is indicated in the Examples. Melting peaks may be generated from melt curve data by the LightCycler 480 analysis function (-dFldt). Tms are calculated by using a manual Tm option to identify the lowest point in the inverse melt peak. Where reference is made to a Tm for hybridisation involving part of an oligonucleotide, the relevant Tm is considered to be the Tm that can be determined for a hybridisation using a test oligonucleotide corresponding to the relevant part of the that oligonucleotide. Primer portions having a suitable Tm typically have a sequence length of between 18 and 30 nucleotides.

Typically, the first oligonucleotide primer (forward primer) has a 5' upstream target-specific portion with respect to the fluorescence cassette-specific portion. In certain embodiments, the first oligonucleotide primer (forward primer) may have a 5' upstream self-annealing portion with respect to the fluorescence cassette-specific portion. By self-annealing is intended that the portion forms a hairpin structure at the Ta of the reaction, such as by having a 5' portion which is the reverse complement to its 3' portion. Typically, the polynucleotide amplification method involving the first oligonucleotide primer is not Polymerase Spiral Reaction, as described in Liu et al, 2015, Sci Rep. 5:12723. doi: 10.1038/srep12723, Polymerase Spiral Reaction (PSR): A novel isothermal nucleic acid amplification method.

In one embodiment, the first oligonucleotide primer (forward primer) has a 5' upstream target-specific portion with respect to the fluorescence cassette-specific portion. Oligonucleotide primer sets including such a first oligonucleotide primer may suitably be applied in loop-mediated isothermal amplification (LAMP), cross-priming amplification (CPA) or smart amplification (SMAP).

A loop-mediated isothermal amplification (LAMP) reaction, as described in Notomi *et al,* 2000, supra requires two inner primers and two outer primers. The inner primers are called the forward inner primer (FIP) and the backward inner primer (BIP) and each contains two target-specific portions corresponding to the sense and antisense sequences of the target DNA, one for priming the first stage and the other for self-priming in later stages. The extension of the outer primers in the first stages causes the extension product of the respective inner primer to be displaced by the strand displacement polymerase. In subsequent stages, the forward primers can hybridise to a single-stranded loop structure which is formed by the hybridisation of the 5' upstream target-specific portion to its complement in the extension product. Further primers may be included to accelerate the reaction, as described in Nagamine *et al,* 2002, *supra.* In an embodiment, the FIP and/or BIP primer may be modified to include the fluorescence cassette-specific portion between the 5' upstream and 3' downstream target-specific portions.

In "single" and "double" cross priming amplification (CPA) described in Xu *et al,* 2012, *supra,* either or both ends of the amplification product respectively include a loop formed by a cross primer. In "single" CPA, the cross primer contains 5' tail sequences which is identical to a primer for the complementary strand. It allows for strand displacement by extension from an upstream primer, and incorporates a second defined priming site in the 5' end of the resulting product. Primers for the opposite strand each consist of a single sequence complementary to the template, and are chosen so that they bind in tandem to the template, providing a region of nicked double stranded DNA which can be extended by a strand displacing DNA polymerase. In a "double" CPA, forward and reverse antisense primers are used, and each cross primer contains 5' tail sequences identical to each other's priming site, thereby introducing additional priming sites in each round of extension. In an embodiment, the cross primer in "single" CPA, or one or both cross primers in "double" CPA may be modified to include the fluorescence cassette-specific portion between the 5' upstream and 3' downstream target-specific portions.

In smart amplification (SMAP) described in Lezhava and Hayashizaki, 2009, *supra,* a tailed turn-back primer (TP) and a tailed folding primer (FP) prime extension reactions from either strand, and are themselves displaced by outer primers. A boost primer is typically also included. The outer primers and the TP may respectively be the same as the outer primers and FIP or BIP used in LAMP. However, the FP differs from FIP or BIP in that its tail is not a target-specific portion, but is a self-annealing portion, which can exist as a hairpin. This facilitates self-primed DNA synthesis from the FP, thus perpetuating the reaction. In particular, when a complement to the folding section is generated, the 3'-end of the synthesis product self-hybridises and acts as a primer to synthesise an additional strand of the target. In an embodiment, the TP may be modified to include the fluorescence cassette-specific portion between the 5' upstream and 3' downstream target-specific portions.

In an alternative embodiment, the first oligonucleotide primer (forward primer) has a 5' upstream self-annealing portion with respect to the fluorescence cassette-specific portion. Oligonucleotide primer sets including such a first oligonucleotide primer may suitably be applied in SMAP. This embodiment corresponds to SMAP in which the FP is modified to include the fluorescence cassette-specific portion.

Given that the first oligonucleotide primer may contain one or two primer portions in addition to the fluorescence cassette-specific portion, it may typically be between 30 and 120 nucleotides in length, such as between 40 and 90 nucleotides in length, or between 40 and 80 nucleotides in length.

Primers are suitably included in the amplification reaction, such as in LAMP, CPA or SMAP, at a concentration range of from 0.1 - 2.0 µM. For LAMP reactions, preferred concentrations are: BIP/FIP primers at 0.8 - 2.0 µM; F31B3 primers at 0.2 - 0.8 µM; and LoopF/LoopB (if present) at 0.4 -1.0 µM. Exemplary primers concentrations and reaction conditions are described in Howard *et al,* 2015, *supra,* for each of LAMP, CPA and SMAP.

### Cassette oligonucleotide sets and fluorescence cassette-specific portions

As noted above, the fluorescence cassette-specific portion or "tag" sequence of each of the first oligonucleotide primers (forward primers) in a particular group may typically be identical or closely related, for example be the same length or differ in length by less than about 10, more typically 5, 4, 3, 2 or 1 nucleotides and there may be no more than 3, 2 or 1 non-identical nucleotides. It may be most straightforward for these fluorescence cassette-specific portions to be identical, but it is not essential, which allows more flexibility in oligonucleotide design.

The fluorescence cassette-specific portion or tag sequence or sequences of one group typically will differ significantly from those of a different group, so that there is no practically relevant hybridisation between the fluorescence cassette-specific portion(s) of one group and the complements of the fluorescence cassette-specific portion(s) of another group, as will be apparent to those skilled in the art.

Suitable cassette oligonucleotide sets and fluorescence cassette-specific portions are described in US 2007/117108 A1, US 2013/252238 A1 and WO 2014/135872 A1.

The first oligonucleotide primers (forward primers) in a group each have a (typically identical or closely related) fluorescence cassette-specific portion. Upon introduction (for example by the progress of the primer extension reaction when the target nucleic acid to which the primers of a particular primer set are directed is present) of a complementary sequence to the fluorescence cassette-specific portion (to which the fluorescent labelled fluorescence cassette oligonucleotide is able to hybridise), a detectable signal is generated, because the acceptor (quencher) labelled fluorescence cassette oligonucleotide is less able to bind to and quench the signal from the fluorescent (donor) labelled fluorescence cassette oligonucleotide. As the polynucleotide amplification reaction progresses and more of the extension product to which the fluorescent labelled fluorescence cassette oligonucleotide is able to hybridise is generated, generally the greater the signal produced.

Thus, in addition to the fluorophore domain, the fluorescent labelled oligonucleotide also comprises a sequence that is capable of hybridisation to the complement of the fluorescence cassette-specific portion of a given group of first oligonucleotide primers (forward primers), This "tag" sequence binds to a nucleic acid sequence (extension product) which is created as a complement to the tagged primer or primers included in the reaction (which may be unlabelled, or which may e.g. in subsequent rounds of the primer extension reaction, be the fluorescent labelled cassette oligonucleotide itself), e.g. under stringent hybridisation conditions. The fluorescent labelled oligonucleotide may or may not be capable of acting as a primer in polynucleotide amplification reaction (for example may have a 3' OH group). It is considered that generally more primer extension product is formed, and hence a better signal obtained, if the or a first cassette oligonucleotide labelled with a fluorescent moiety is capable of acting as a primer in the polynucleotide amplification reaction. The acceptor/quencher labelled fluorescence cassette oligonucleotide may typically not be capable of acting as a primer in a primer extension reaction, for example because the acceptor/quencher may prevent the oligonucleotide from acting as a primer.

In addition to the acceptor domain, the acceptor moiety labelled cassette oligonucleotide is capable of hybridising to the corresponding fluorescent labelled cassette oligonucleotide to form a fluorescent quenched pair.

Typically the fluorescent labelled cassette oligonucleotide does not comprise a target sequence specific portion i.e. does not comprise a sequence hybridising (at the Ta of the polynucleotide amplification reaction) to the target polynucleotide that a target specific portion of the first oligonucleotide primer or primers is intended to hybridise with. Thus, in such an arrangement, the fluorescent labelled cassette oligonucleotide is not tied to a particular target sequence but may be used (with appropriate oligonucleotide primer sets) in the detection of a polynucleotide amplification product, arising from any target sequence.

The fluorescent labelled cassette oligonucleotide (and corresponding acceptor/quencher labelled cassette oligonucleotide) may be included in a "master" assay mix, to be used alongside an (target specific) "assay" mix. Typically, the acceptor/donor labelled cassette oligonucleotide does not comprise a target sequence specific portion either. Thus, in an embodiment, the first and second cassette oligonucleotides in each set do not comprise a target-specific portion.

In an alternative arrangement, the first oligonucleotide primer is the fluorescent labelled cassette oligonucleotide, and the fluorescent label is directly incorporated into the amplification product.

Typically the fluorescent labelled cassette oligonucleotide consists of the fluorescent moiety (donor moiety) and the sequence that is capable of hybridisation to the complement of the fluorescence cassette-specific portion of a first oligonucleotide primer. Typically the acceptor/quencher labelled cassette oligonucleotide consists of the acceptor/quencher moiety and the sequence that is capable of hybridisation to the fluorescence cassette-specific portion of the fluorescent labelled cassette oligonucleotide.

The interaction between the fluorescent (donor) labelled fluorescence cassette oligonucleotide and the acceptor (quencher) labelled fluorescence cassette oligonucleotide is typically less stable than the interaction between the fluorescent (donor) labelled fluorescence cassette oligonucleotide and the extension product complementary to the fluorescence cassette-specific portion of the forward oligonucleotide primer of each primer set of the relevant group. Such an arrangement may be useful in achieving an optimal balance between avoiding generation of aberrant signal and allowing the primer extension reaction to proceed efficiently. Thus, the Tm for the hybridisation between the fluorescent (donor) labelled fluorescence cassette oligonucleotide and the acceptor (quencher) labelled fluorescence cassette oligonucleotide (herein Tm A) may be lower than the Tm for the hybridisation between the fluorescent (donor) labelled fluorescence cassette oligonucleotide and the extension product complementary to the fluorescence cassette-specific portion of the forward oligonucleotide primer (herein Tm C) of each primer set of the relevant group (for example between about 1 and 10 °C lower).

In an example (for example when the fluorescent labelled cassette oligonucleotide does not comprise a target-specific sequence), the quencher labelled cassette oligonucleotide is between 1 and 5 nucleotide bases shorter than the fluorescent labelled cassette oligonucleotide, Typically the unpaired (relative to the quencher labelled cassette oligonucleotide) portion of the fluorescent labelled cassette oligonucleotide is at the 3' end of the fluorescent labelled cassette oligonucleotide or "opposite" the 5' end of the quencher labelled cassette oligonucleotide. The portion of the relevant oligonucleotide primer (or primers) whose complement hybridises to the fluorescent labelled cassette oligonucleotide typically is within about 5 nucleotides of the length (for example between 5, 4, 3, 2, or 1 nucleotides shorter and 5, 4, 3, 2, or 1 nucleotides longer; for example the same length) of the fluorescence labelled cassette oligonucleotide, for example with any difference in length typically at the 5' end of the oligonucleotide primer and fluorescence labelled cassette oligonucleotide. In subsequent rounds of the polynucleotide amplification reaction, the fluorescent labelled cassette oligonucleotide itself can act as a primer, so the complement formed during primer extension will generally extend to the 5' end of the fluorescent labelled cassette oligonucleotide. Thus, the portion of the complement that hybridises to the quencher labelled cassette oligonucleotide is typically as long as the quencher labelled cassette oligonucleotide.

In other examples, there may alternatively or in addition be more (or more significant) mismatches between the quencher labelled cassette oligonucleotide and the fluorescent labelled cassette oligonucleotide than between the complement to the relevant oligonucleotide primer (or primers) and the fluorescent labelled cassette oligonucleotide. As is well known to those skilled in the art, the position of a mismatch within an oligonucleotide pair and the nature of the mismatch (for example whether an A:T pairing is disrupted or a G:C pairing) will influence the significance of the mismatch on the change in stability/change in Tm.

In yet further examples, alternatively or in addition different nucleotides/bases may be used in the quencher labelled cassette oligonucleotide relative to those used in the primer extension reaction mix (and hence incorporated into the complement to the primers) thereby altering the relative stability of the hybridisations between the quencher labelled cassette oligonucleotide and the fluorescent labelled cassette oligonucleotide and primer extension product. Typically the "non-standard" base or bases may be used in the quencher labelled cassette oligonucleotide, and "standard" bases in the primer extension mix, but other arrangements are also possible, as will be apparent to the skilled person.

The Tm (Tm A) of the fluorescent quenched pair or pairs is typically within (±) 20°C of, such as within (±)10°C of the Ta of the isothermal polynucleotide amplification reaction. In exemplary embodiments, the Tm of the fluorescent quenched pair may be about 55 °C, as described in US 2007/117108 A1, between 48 and 50 °C, as described in US 2013/252238 A1, or about 62 °C, as described in WO 2014/135872 A1.

In an embodiment, the Tm (Tm A) of the fluorescent quenched pair or pairs is above the Ta of the isothermal polynucleotide amplification reaction, such as up to 15°C, up to 10°C, or between 1 and 10°C above the Ta of the isothermal polynucleotide amplification reaction. In this arrangement, unincorporated fluorescent oligonucleotide is hybridised to the quencher oligonucleotide at the fluorescence acquisition temperature allowing the reaction to be monitored in real-time or at end point. An advantage of this embodiment is that real-time detection may be performed at the temperature of the amplification reaction; cooling of the reaction is not required. Suitable fluorescent quenched pairs and fluorescence cassette-specific oligonucleotide portions are described in WO 2014/135872 A1.

Independently of whether the Tm A is above, at or below the Ta, one of the first or second oligonucleotide cassettes in each set may have a Tm that is at or below the Ta of the isothermal polynucleotide amplification reaction. Typically, the Tm of the second oligonucleotide cassette is at or below the Ta, and the Tm of the first cassette oligonucleotide is above the Ta of the reaction. In this arrangement, the single-labelled oligonucleotide sequences have differing Tms and the one with the greater Tm is typically more than 10 nucleotides longer than the other and preferably at least 15 nucleotides longer. Suitable fluorescent quenched pairs and fluorescence cassette-specific oligonucleotide portions are described in US 2007/117108 A1 and US 2013/252238 A1.

In the present invention, one or both of the pair of labelled fluorescent cassette oligonucleotides (or the primer oligonucleotides) may contain modified bases such as phosphorothioate-modified bases. The number of phosphodiester linkages replaced by phosphorothioates in any given oligonucleotide/primer can range from none to all of the phosphodiester bonds being replaced by phosphothioates, for example one, two, three, four or more. The oligonucleotide(s)/primer(s) may contain phosphorothioates at the 5' and/or 3' termini, however it is preferred that, as an alternative to or addition to such terminal modifications, at least one of the internal bases of the oligonucleotide/primer is a phosphorothioate. For example 10-90%, 20-80%, 30-70% or 40-60% of the bases may be phosphorothioates. In one embodiment the phosphorothioate-modified bases (where there is more than one) are separated by at least one, e.g. one to three, unmodified (phosphorodiester) bases, for example alternate bases within the oligonucleotide(s)/primer(s) may be phosphorothioates. In an example, it may be particularly useful for the fluorescent (donor) labelled fluorescent cassette oligonucleotide or oligonucleotides to contain phosphorothioate-modified bases. It is considered that the presence of phosphorothioate-modified bases may assist in reducing the formation of aberrant products that may be fluorescent and therefore lead to generation of erroneous fluorescence signal. See, for example, US 2013/0252238, for discussion of phosphorothioate incorporation patterns that are considered also to be useful in relation to the present invention.

### Signal detection

Fluorescent energy transfer occurs when a suitable fluorescent energy donor and an energy acceptor moiety are in close proximity to one another. The excitation energy absorbed by the donor is transferred to the acceptor which can then further dissipate this energy either by fluorescent emission if a fluorophore, or by non-fluorescent means if a quencher. A donor-acceptor pair comprises two - a fluorescence group and a fluorescence-modifying group having overlapping spectra, where the donor (fluorescence group) emission overlaps the acceptor (fluorescence-modifying) absorption, so that there is energy transfer from the excited fluorophore to the other member of the pair. As such, the labelled oligonucleotides pair(s) (fluorescence cassette oligonucleotide sets) of the invention are nucleic acid detectors that include on separate oligonucleotides a fluorophore domain where the fluorescent energy donor, i.e. donor, is positioned and a second oligonucleotide with an acceptor domain where the fluorescent energy acceptor, i.e. acceptor, is positioned. As mentioned above, the donor oligonucleotide includes the donor fluorophore. The donor fluorophore may be positioned anywhere in the nucleic acid detector, but is typically present at the 5' end of the oligonucleotide.

The acceptor domain includes the fluorescence energy acceptor. The acceptor may be positioned anywhere in the acceptor domain, but is typically present at the 3' end of the oligonucleotide.

In the present invention, in a pair of labelled oligonucleotides each of the cassette oligonucleotides may contain one or more labels, for example 1, 2 or 3 labels. One or both of the cassette oligonucleotides preferably contains a single label, more preferably both of the oligonucleotides contain a single label.

For example the fluorescent labelled cassette oligonucleotide preferably contains a label at or within the 5' end of the oligonucleotide and the quencher labelled cassette oligonucleotide contains a label at or within the 3' end of the oligonucleotide.

The fluorophores for the labelled oligonucleotide pairs may be selected so as to be from a similar chemical family or a different one, such as cyanine dyes, xanthenes or the like. Fluorophores of interest include, but are not limited to fluorescein dyes (e.g. 5-carboxyfluorescein (5-FAM), 6-carboxyfluorescein (6-FAM), 2',4',1,4,-tetrachlorofluorescein (TET), 2',4',5',7',1,4-hexachlorofluorescein (HEX), and 2',7'-dimethoxy-4',5'-dichloro-6-carboxyfluorescein (JOE)), cyanine dyes such as Cy5, dansyl derivatives, rhodamine dyes (e.g. tetramethyl-6-carboxyrhodamine (TAMRA), and tetrapropano-6-carboxyrhodamine (ROX)), DABSYL, DABCYL, cyanine, such as Cy3, anthraquinone, nitrothiazole, and nitroimidazole compounds, or other non-intercalating dyes. Fluorophores of interest are further described in International Patent Applications WO 01/42505 and WO 01/86001.

If more than one primer groups are used (for example 1, 2, 3, 4, 5, 6, 7, 8, 9, or 10) and consequently several cassette oligonucleotide sets, the fluorophore group may typically be different for each of the 1, 2, 3, 4, 5, 6, 7, 8, 9 or 10 (or more) cassette oligonucleotide sets. The acceptor (for example quencher) group may be the same or different so long as they are able to modulate the fluorescence of the paired fluorophore group in satisfactory fashion. Typically 1, 2, 3, or 4 different primer groups (each of which may have one or more primer sets, typically one primer set) and consequently 1, 2, 3 or 4 cassette oligonucleotide sets may be used in a single reaction tube. A limiting factor may be the number of different spectra that it is possible to distinguish, which may depend on the characteristics of the fluorescence generating/measuring equipment available. Considerations in choosing compatible sets of fluorophores and acceptors/quenchers will be known to those skilled in the art.

The signal that is generated in the methods of the invention may be detected by measuring the signal at any point during or after the polynucleotide amplification reaction. Measurement of the signal may be qualitative or quantitative. The signal may be detected at the end point of the reaction, or it may be detected in real-time. Where the Tm of the fluorescent quenched pair is below the Ta of the amplification reaction, the reaction may be periodically cooled to permit measurement, such as to a temperature at or below the Tm of the fluorescent quenched pair. Typically, the temperature is only cooled to the degree necessary to detect the fluorescence. For example, the reaction may be cooled to 37 °C every five minutes.

The ratio of the acceptor labelled cassette oligonucleotide relative to fluorophore labelled cassette oligonucleotide may be varied in the polynucleotide amplification reaction to optimise the signal achieved and/or minimise the signal arising in a "no template control" (NTC). Suitable ratios may be at least 0.75:1, 1:1, 1.5:1, 2:1, 3:1, 4:1 or 5:1, for example between 1:1 and 10:1, for example between 1:1 and 5:1 acceptor-labelled to fluorophore-labelled cassette oligonucleotide in a cassette oligonucleotide set.

### Applications

The present invention finds use in a variety of different applications, and is particularly suited for use for use in allele specific SNP Genotyping, somatic mutation detection, pathogen detection, gene expression studies or copy number variation studies, and the like.

### Definitions

As used herein, "nucleic acid" means either DNA, RNA, single-stranded or double-stranded, and any chemical modifications thereof. Modifications include, but are not limited to, those which provide other chemical groups that incorporate additional charge, polarisability, hydrogen bonding, electrostatic interaction, and functionality to the nucleic acid. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases isocytidine and isoguanidine and the like. Modifications can also include 3' and 5' modifications such as capping.

As used herein, "complementary" refers to the pair of nitrogenous bases, consisting of a purine linked by hydrogen bonds to a pyrimidine, that connects the complementary strands of DNA or of hybrid molecules joining DNA and RNA.

As used herein, "fluorescent group" refers to a molecule that, when excited with light having a selected wavelength, emits light of a different wavelength. Fluorescent groups may also be referred to as "fluorophores".

As used herein, "fluorescence-modifying group" refers to a molecule that can alter in any way the fluorescence emission from a fluorescent group. A fluorescence-modifying group generally accomplishes this through an energy transfer mechanism. Depending on the identity of the fluorescence-modifying group, the fluorescence emission can undergo a number of alterations, including, but not limited to, attenuation, complete quenching, enhancement, a shift in wavelength, a shift in polarity, a change in fluorescence lifetime. One example of a fluorescence-modifying group is a quenching group.

As used herein, "energy transfer" refers to the process by which the fluorescence emission of a fluorescent group is altered by a fluorescence-modifying group. If the fluorescence-modifying group is a quenching group, then the fluorescence emission from the fluorescent group is attenuated (quenched). Energy transfer can occur through fluorescence resonance energy transfer, or through direct energy transfer. The exact energy transfer mechanisms in these two cases are different. It is to be understood that any reference to energy transfer in the present application encompasses all of these mechanistically-distinct phenomena. Energy transfer is also referred to herein as fluorescent energy transfer or FET.

As used herein, "energy transfer pair" refers to any two molecules that participate in energy transfer. Typically, one of the molecules acts as a fluorescent group, and the other acts as a fluorescence-modifying group. Such pairs may comprise, for example, two fluorescent groups which may be different from one another and one quenching group, two quenching groups and one fluorescent group, or multiple fluorescent groups and multiple quenching groups. In cases where there are multiple fluorescent groups and/or multiple quenching groups, the individual fluorescent and/or quenching groups may be different from one another. The preferred energy transfer pairs of the disclosure comprise a fluorescent group and a quenching group. In some cases, the distinction between the fluorescent group and the fluorescence-modifying group may be blurred. For example, under certain circumstances, two adjacent fluorescein groups can quench one another's fluorescence emission via direct energy transfer. For this reason, there is no limitation on the identity of the individual members of the energy transfer pair in this application. All that is required is that the spectroscopic properties of the energy transfer pair as a whole change in some measurable way if the distance between the individual members is altered by some critical amount.

As used herein, "primer" refers to an oligonucleotide which is capable of acting as a point of initiation of synthesis when placed under conditions in which synthesis of a primer extension product which is complementary to a nucleic acid strand can occur. Thus, an oligonucleotide capable of acting as a primer may typically have a 3' OH group.

As used herein, "quenching group" refers to any fluorescence-modifying group that can attenuate at least partly the light emitted by a fluorescent group. We refer herein to this attenuation as "quenching". Hence, illumination of the fluorescent group in the presence of the quenching group leads to an emission signal that is less intense than expected, or even completely absent. Quenching occurs through energy transfer between the fluorescent group and the quenching group.

As used herein, "fluorescence resonance energy transfer" or "FRET" refers to an energy transfer phenomenon in which the light emitted by the excited fluorescent group is absorbed at least partially by a fluorescence-modifying group. If the fluorescence-modifying group is a quenching group, then that group can either radiate the absorbed light as light of a different wavelength, or it can dissipate it as heat. FRET depends on an overlap between the emission spectrum of the fluorescent group and the absorption spectrum of the quenching group. FRET also depends on the distance between the quenching group and the fluorescent group. Above a certain critical distance, the quenching group is unable to absorb the light emitted by the fluorescent group, or can do so only poorly.

As used herein "tailed primer" refers to an oligonucleotide containing at least two domains, one specific to the target nucleic acid, e.g. DNA, of interest, i.e. capable of hybridising to said target nucleic acid, and the other sequence (typically 5' of the target-specific sequence), serving as a template for production of extension product comprising the complement of the "tag" sequence. The complement of the "tag" sequence may then bind to, for example, the "tag" portion of the corresponding fluorescent labelled cassette oligonucleotide (which may lack any sequence other than "tag" portion). The tagged primers may also comprise additional regions such as a linker between the two domains referred to above and/or tags.

As used herein "direct energy transfer" refers to an energy transfer mechanism in which passage of a photon between the fluorescent group and the fluorescence-modifying group does not occur. Without being bound by a single mechanism, it is believed that in direct energy transfer, the fluorescent group and the fluorescence-modifying group interfere with each other's electronic structure. If the fluorescence-modifying group is a quenching group, this will result in the quenching group preventing the fluorescent group from emitting light.

In general, quenching by direct energy transfer is more efficient than quenching by FRET. Indeed, some quenching groups that do not quench particular fluorescent groups by FRET (because they do not have the necessary spectral overlap with the fluorescent group) can do so efficiently by direct energy transfer. Furthermore, some fluorescent groups can act as quenching groups themselves if they are close enough to other fluorescent groups to cause direct energy transfer. For example, under these conditions, two adjacent fluorescein groups can quench one another's fluorescence effectively. For these reasons, there is no limitation on the nature of the fluorescent groups and quenching groups useful for the practice of this invention.

Where reference is made to "hybridisation" or the ability of an oligonucleotide and/or primer to "hybridise" to another nucleotide sequence, the skilled person will understand that such hybridisation is capable of occurring under the conditions prevalent in the polynucleotide amplification reaction in which the oligonucleotide and/or primer is utilised.

### Kits

The invention also provides a kit suitable for use in a method for the detection of one or more polynucleotide amplification products, the kit comprising:
i) a first oligonucleotide primer (forward primer) comprising a 5' upstream target-specific or self-annealing portion, a fluorescence cassette-specific portion and a 3' downstream target-specific portion; and
ii) one or more target-specific oligonucleotide primers;

wherein the oligonucleotide primers in a particular set are each suitable for hybridisation to a strand of a target polynucleotide to permit formation of an amplification product of the target polynucleotide in an isothermal polynucleotide amplification reaction;
and wherein the first oligonucleotide primer of each set in the same group contains a fluorescence cassette-specific portion that is capable of hybridising to the complement of the fluorescence cassette-specific portion of the first oligonucleotide primer of any set in the same group;

b) one or more cassette oligonucleotide sets, each set characterised by:
   i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
   ii) a second cassette oligonucleotide labelled with an acceptor moiety (for example a quencher moiety);
wherein the cassette oligonucleotides of each set hybridise to one another to form a fluorescent quenched pair.

The invention also provides a kit suitable for use in a method for the detection of one or more polynucleotide amplification products, the kit comprising:
i) a first oligonucleotide primer (forward primer) comprising a fluorescence cassette-specific portion and a 3' downstream target-specific portion; and
ii) one or more target-specific oligonucleotide primers;

wherein the oligonucleotide primers in a particular set are each suitable for hybridisation to a strand of a target polynucleotide to permit formation of an amplification product of the target polynucleotide in an isothermal polynucleotide amplification reaction;
and wherein the first oligonucleotide primer of each set in the same group contains a fluorescence cassette-specific portion that is capable of hybridising to the complement of the fluorescence cassette-specific portion of the first oligonucleotide primer of any set in the same group;

b) one or more cassette oligonucleotide sets, each set characterised by:
   i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
   ii) a second cassette oligonucleotide labelled with an acceptor moiety (for example a quencher moiety);
wherein the cassette oligonucleotides of each set hybridise to one another to form a fluorescent quenched pair; and
c) a strand-displacement polymerase.

In either kit, the first oligonucleotide primers may typically be unlabelled. The fluorescent labelled cassette oligonucleotide typically does not comprise a target-specific sequence.

Further preferences for the oligonucleotides are as indicated above.

The kits according to the disclosure may also contain other components suitable for use in a polynucleotide amplification reaction such as divalent cations, e.g. derived from magnesium salts, deoxyribonucleotide 5' triphosphates (dNTPs), buffering agents, etc.

The kit may comprise the one or more cassette oligonucleotide sets in a first container, optionally wherein the first container comprises other components for performing a polynucleotide amplification reaction, such as buffer, strand-displacement polymerase, and optionally wherein the first cassette oligonucleotides do not comprise a target-specific portion; and one or more oligonucleotide primer groups in a separate further container or containers.

### Indirect (real-time) detection of polynucleotide amplification products

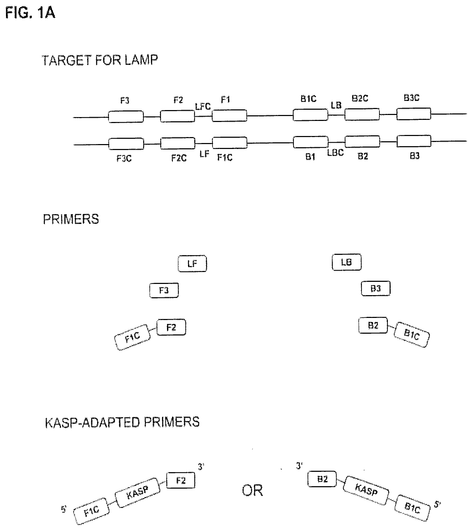

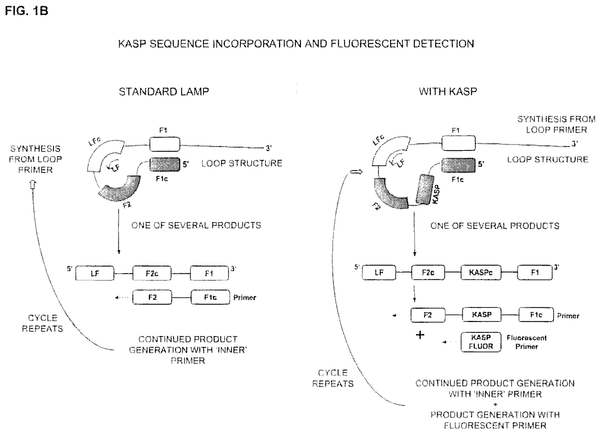

This embodiment utilises unlabelled oligonucleotide primers to initiate a LAMP reaction. The FIP or BIP primer is modified by the introduction of a "tag" DNA sequence between the 5' upstream and 3' downstream target-specific sequences, and is used together with the other five LAMP primers, as illustrated in Figure 1. Also included in the reaction is a single fluorescently-labelled oligonucleotide that is capable of hybridising to the complement of the tag sequence region of the modified FIP or BIP primer generated in the reaction. A number of suitable fluorophores exist, with a popular choice being FAM (a derivative of fluorescein). Finally, included in the reaction is a 3' quencher-labelled oligonucleotide antisense to the FAM labelled oligonucleotide. A number of suitable labels exist of which the Black Hole quencher series of labels are a popular choice.

As the length of the quencher oligonucleotide is long enough to give a Tm above the Ta of the reaction the product generation can be assessed in real-time (such as using a Roche LC480 or ABI 7900 Prism instrument).

Due to the complementarity of the two labelled oligonucleotides, they hybridise to each other. This hybridisation brings the quencher label in very close proximity to the fluorophore, thereby rendering all fluorescent signal from the fluorophore quenched. The polynucleotide amplification process is initiated and amplification product begins to be generated. After the first few rounds of amplification, the complementary sequence to the tagged oligonucleotide is generated. The fluorescent oligonucleotide is then able to initiate synthesis during the polynucleotide amplification reaction, and does so. It is not essential that the fluorescent oligonucleotide is able to act as a primer, but it is considered that more product may be generated if the fluorescent oligonucleotide acts as a primer, which may provide a better signal. This produces an amplicon containing a fluorescent molecule. Once this occurs the quenching oligo is less able to hybridise to the fluorescent labelled oligonucleotide, as the amplification process produces double-stranded amplicon DNA. As the quenching oligonucleotide is no longer hybridised to the fluorescent labelled oligonucleotide, signal is then generated which is directly proportional to the amount of amplification product generated and can be measured.

The tag region of the modified FIP or BIP primer need not be identical to the single fluorescently-labelled oligonucleotide, as long as a complementary sequence of the tag region generated hybridises to the fluorescently-labelled oligonucleotide.

### Indirect (end-point) Detection of polynucleotide amplification products - SNP Genotyping

This embodiment utilises the same fluorophore- and quencher-labelled oligonucleotide pair(s) as described above.

SNP genotyping utilises at least two labelled oligonucleotide pairs, for example 2, 3 or 4 pairs, wherein each pair preferably comprises a different fluorophore, which fluorophores are spectrally-resolvable from each other, e.g. FAM and HEX. The tagged primers (each corresponding to a different oligonucleotide primer group, as indicated above) are tagged with a distinct sequence, the non-tagged portions of the primers (generally termed forward) are directed to the DNA of interest. In the 3' target-specific portion of the primer they may differ from one another only by a single nucleotide e.g. at their 3' terminal base. Each primer is directed to the polymorphic base in the DNA of interest, as well known to those skilled in the art. Primer extension is conducted whereby the primers only initiate synthesis when they match the target sequence of interest, e.g. when the 3' base is perfectly matched. When a mismatch occurs, synthesis does not proceed.

During the reaction, the 3' target specific portion depending on the genotype is able to initiate synthesis (or both are, in the case of a heterozygote). This results in incorporation of the distinct tagged portion of the primer into the polynucleotide amplification product thereby hindering the hybridisation of the quencher oligonucleotide to the corresponding fluorescent oligonucleotide. Signal is therefore generated according to which of the allele-specific oligonucleotides has initiated the synthesis. The amplification products incorporating one or more of the fluorophores may then be read on a fluorescent plate-reader. The resulting data may then be plotted and a cluster plot of one fluorophore over the other is generated. The resulting genotypes are then able to be determined based on the cluster plots.

In this specification and the appended claims, the singular forms "a", "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the ranges, and are also encompassed within the invention, subject to any specifically excluded limit in the stated range. Where the stated range includes one or both of the limits, ranges excluding either or both of those included limits are also included in the invention.

The present invention will be further illustrated in the following examples, without any limitation thereto.

### Example 1: Mitochondrial SNP genotyping in a LAMP reaction

As mitochondrial DNA is homozygous, individual samples possess only one allele of a SNP, allowing for detection of individual allele-specific amplification products in a multiplex reaction. A mitochondrial SNP genotyping assay (human mtDNA 11719) was used to demonstrate proof-of-concept for universal detection using a LAMP assay.

The mitochondrial DNA was extracted from human blood and the relevant target portion has the following DNA sequence, in which the SNP is shown in bold:

ACAAAACACATAGCCTACCCCTTCCTTGTACTATCCCTATGAGGCATAATTATAACAA
GCTCCATCTGCCTACGACAAACAGACCTAAAATCGCTCATTGCATACTCTTCAATCA
GCCACATAGCCCTCGTAGTAACAGCCATTCTCATCCAAACCCCCTGAAGCTTCACC
GGCGCAGTCATTCTCATAATCGCCCACGG[A/G]CTTACATCCTCATTACTATTCTGC
CTAGCAAACTCAAACTACGAACGCACTCACAGTCGCATCATAATCCTCTCTCAAGGA
CTTCAAACTCTACTCCCACTAATAGCTTTTTGATGACTTCTAGCAAGCCTCGCTAACC
TCGCCTTACCCCCCACTATTAACCTACTGGGAGAACTCTCTGTGCTAGTAACCACGT
TCTC

Reverse (BIP) LAMP primers BIPKASP AI1 and BIPKASP AI2 were designed to incorporate a universal tag sequence identical to a portion of a FAM (fluorescein) or HEX-labelled cassette oligonucleotide sequence respectively, and to include a 3' nucleotide corresponding to the allele to be detected. LAMP primers are shown in the table below, with tag sequences underlined and the SNP sequence shown in bold.

**Table 1: Primers for mtDNA11719 LAMP assay.**

| | |
|---|---|
| 161-6 FIP | GGATGAGAATGGCTGTTACTACG GACAAACAGACCTAAAATCGCTC |
| 161-6 F3 | GCATAATTATAACAAGCTCCATCTGC |
| 161-6 B3 | CTGTGAGTGCGTTCGTAGTTTGAG |
| 161-6 LF | GCTATGTGGCTGATTGAAGAGTATG |
| 161-6 LB | CATTCTCATAATCGCCCACG |
| 161-6 BIPKASP AI1 | GAAGCTTCACCGGCGCAG GAAGGTGACCAAGTTCATGCTCTAGGCAGAATAGTAATGAGGATGTAAG C |
| 161-6 BIPKASP AI2 | GAAGCTTCACCGGCGCAG GAAGGTCGGAGTCAACGGATTCTAGGCAGAATAGTAATGAGGATGTAA GT |

Primers were combined into a bulk mix as follows:

**Table 2: Bulk mix made to produce a 250 µl reaction volume of LAMP oligonucleotides.**

| Oligonucleotide | Starting concentration | Volume added |
|---|---|---|
| 161-6 FIP | 100µM | 75µl |
| 161-6 F3 | 100µM | 25µl |
| 161-6 B3 | 100µM | 25µl |
| 161-6 LF | 100µM | 50µl |
| 161-6 LB | 100µM | 50µl |
| 161-6 BIPKASP AI1 | 100µM | 37.5µl |
| 161-6 BIPKASP AI2 | 100µM | 37.5µl |
| Ultrapure water | Neat | 637.5µl |

Cassette oligonucleotide sequences are shown below:

**Table 3: Cassette oligonucleotide sequences.**

| | |
|---|---|
| FAM fluor | 156FAMITC AGA AGG TGA CCA AGT TCA TGC |
| HEX fluor | /5HEX/AC TGA AGG TCG GAG TCA ACG GA |
| FAM quencher | CAT GAA CTT GGT CAC CTT CGG A/3Dab/ |
| HEX quencher | CGT TGA CTC CGA CCT TCA G/3Dab/ |

A 20x cassette mix was made as follows:

**Table 4: Cassette mix.**

| Oligonucleotide | Starting concentration | Volume added |
|---|---|---|
| FAM fluor | 100µM | 11.6µl |
| HEX fluor | 100µM | 11.6µl |
| FAM quencher | 500µM | 18.93µl |
| HEX quencher | 500µM | 18.93µl |
| TrisIEDTA pH 8.3 | 10mM Tris, 0.1mM EDTA | 938.94µl |

Isothermal Master Mix, comprising strand-displacement DNA polymerase, inorganic pyrophosphatase, reaction buffer, MgSO₄ and dNTPS was purchased (OptiGene Ltd, UK, Cat# ISO-001 nd) and reagents combined (per reaction) as follows:

**Table 5: Reaction mix.**

| Reagent | Concentration | Volume |
|---|---|---|
| Isothermal Master Mix | As supplied, ~1.7x | 15µl |
| Primer Mix | As above | 3.75µl |
| Fluor/Quencher Mix | 20x | 1.25µl |
| Sample | ~5ng/µl | 5µl |

Amplification was performed in 25µl reactions in a 96-well white plate on a Roche Light Cycler 480 instrument. Amplification was performed at 65°C for 90 minutes with fluorescent detection every minute (one cycle). Following the 90-minute incubation the reaction was cooled to 37°C and a final fluorescent read performed.

Real-time amplification data is displayed in figure 2A (FAM detection) and 2B (HEX detection). These data demonstrate that amplification can be completed within twenty minutes. End point analysis in Figure 2C shows that clustered groups of FAM or HEX fluorescent products are distinguishable even after ninety minutes, reflecting the presence of the different alleles of the mtDNA11719 SNP in the different samples.

### Example 2: Further LAMP reaction for mitochondrial SNP genotyping

The mitochondrial SNP genotyping assay of Example 1 was repeated using a different strand-displacement DNA polymerase.

Primers, as described for Example 1, were combined into a bulk primer mix as follows:

**Table 6: Bulk primer mix.**

| Oligonucleotide | Starting concentration | Volume added |
|---|---|---|
| 161-6 FIP | 100µM | 100µl |
| 161-6 F3 | 100µM | 25µl |
| 161-6 B3 | 100µM | 25µl |
| 161-6 LF | 100µM | 50µl |
| 161-6 LB | 100µM | 50µl |
| 161-6 BIPKASP AI1 | 100µM | 50µl |
| 161-6 BIPKASP AI2 | 100µM | 50µl |
| Ultrapure water | Neat | 775µl |

A 20x cassette mix using the cassette oligonucleotide sequences described in Example 1 was made as follows:

**Table 7: Cassette mix.**

| Oligonucleotide | Starting concentration | Volume added |
|---|---|---|
| FAM fluor | 500µM | 2.32µl |
| HEX fluor | 500µM | 2.32µl |
| FAM quencher | 500µM | 18.93µl |
| HEX quencher | 500µM | 18.93µl |
| Tris/EDTA pH 8.3 | 10mM Tris, 0.1mM EDTA | 938.94µl |

Isothermal Master Mix was prepared as in Table 8 using *Bst* 3.0 DNA polymerase (NEB Cat # M0374) and accompanying reagents purchased from New England Biolabs. The Isothermal Amplification Buffer II (NEB Cat # B0374S) following dilution to 1X as in the final reaction mixture, provides 20 mM Tris-HCl, 10 mM (NH₄)₂SO₄, 150 mM KCI, 2 mM MgSO₄, 0.1 % Tween^{®} 20, (pH 8.8 @ 25°C). Additional MgSO₄ included in the Master Mix brought the final concentration of MgSO₄ to 4 mM, which was the optimal concentration for the reaction. A preferred range for the final concentration of MgSO₄ is 3.5mM - 5.5mM.

**Table 8: Isothermal Master Mix for 25 reactions.**

| Component | Starting concentration | Volume added |
|---|---|---|
| Isothermal Amplification Buffer II | 10X | 62.5µl |
| MgSO₄ | 100mM | 12.5µl |
| *Bst* 3.0 DNA polymerase | 8000U/mL | 25µl |
| dNTPs | 25mM | 35µl |
| Nuclease free water | Neat | 215µl |

Reagents were combined (per 25 µl reaction) as follows:

**Table 9: Reaction mix.**

| Reagent | Concentration | Volume |
|---|---|---|
| Isothermal Master Mix | As above | 14µl |
| Primer Mix | As above | 4.5µl |
| Fluor/Quencher Mix | 20x | 1.5µl |
| Sample | ~5ng/µl | 5µl |

Amplification was performed as in Example 1 and representative results from several experiments are shown in Figure 3A-C.

## Claims

1. A method for the detection of one or more polynucleotide amplification products, the method comprising the steps of:
a) providing one or more oligonucleotide primer groups, each group comprising one or more oligonucleotide primer sets, each set **characterised by**:
i) a first oligonucleotide primer (forward primer) comprising a fluorescence cassette-specific portion and a 3' downstream target-specific portion; and
ii) one or more target-specific oligonucleotide primers;
wherein the oligonucleotide primers in a particular set are each suitable for hybridisation to a strand of a target polynucleotide to permit formation of an amplification product of the target polynucleotide in an isothermal polynucleotide amplification reaction;
and wherein the first oligonucleotide primer of each set in the same group contains a fluorescence cassette-specific portion that is capable of hybridising to the complement of the fluorescence cassette-specific portion of the first oligonucleotide primer of any set in the same group;
b) providing one or more cassette oligonucleotide sets, each set **characterised by**:
i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
ii) a second cassette oligonucleotide labelled with an acceptor moiety (for example a quencher moiety);
wherein the cassette oligonucleotides of each set hybridise to one another to form a fluorescent quenched pair;
c) initiating an isothermal polynucleotide amplification reaction in the presence of a strand displacement DNA polymerase thereby generating (if the target polynucleotide is present) a complementary sequence to at least a portion of the relevant first oligonucleotide primer, such that the relevant second (acceptor, for example quencher, labelled) cassette oligonucleotide is less able to hybridise to the relevant first (fluorescently labelled) cassette oligonucleotide, whereby a signal is generated; and
d) detecting the signal that is generated.

2. The method of Claim 1 wherein the first oligonucleotide primer (forward primer) has a 5' upstream target-specific or self-annealing portion with respect to the fluorescence cassette-specific portion.

3. The method of Claim 2 wherein the first oligonucleotide primer (forward primer) has a 5' upstream target-specific portion with respect to the fluorescence cassette-specific portion, such as wherein the isothermal polynucleotide amplification reaction is loop-mediated isothermal amplification (LAMP), cross-priming amplification (CPA) or smart amplification (SMAP).

4. The method of Claim 2 wherein the first oligonucleotide primer (forward primer) has a 5' upstream self-annealing portion with respect to the fluorescence cassette-specific portion, such as wherein the isothermal polynucleotide amplification reaction is smart amplification (SMAP).

5. The method of any one of the preceding claims wherein the first and second cassette oligonucleotides in each set do not comprise a target-specific portion.

6. The method of any one of the preceding claims wherein the Tm (Tm A) of the fluorescent quenched pair or pairs is within (±) 20°C of, such as within (±)10°C of the Ta of the amplification reaction; optionally
wherein the Tm (Tm A) of the fluorescent quenched pair or pairs is above the Ta of the isothermal polynucleotide amplification reaction, such as up to 15°C, up to 10°C, or between 1 and 10°C above the Ta of the isothermal polynucleotide amplification reaction.

7. The method of any one of the preceding claims wherein one of the first or second oligonucleotide cassettes in each set has a Tm that is at or below the Ta of the isothermal polynucleotide amplification reaction.

8. The method of any one of the preceding claims wherein the signal is detected at the end point of the reaction.

9. The method of any one of Claims 1 to 7 wherein the signal is detected in real-time.

10. The method of any one of the preceding claims wherein at least one of the bases of at least one of the oligonucleotides, for example at least one of the cassette oligonucleotides, optionally the fluorescent labelled cassette oligonucleotide, is a phosphorothioate; optionally
wherein 20-80% of the bases of at least one of the oligonucleotides, for example at least one of the cassette oligonucleotides, optionally the fluorescent labelled cassette oligonucleotide, are phosphorothioates.

11. The method of any one of the preceding claims wherein there are at least 2 and optionally 3, 4, 5, 6, 7, 8, 9 or 10 oligonucleotide primer groups and corresponding cassette oligonucleotide sets.

12. A kit suitable for use in a method for the detection of one or more polynucleotide amplification products, the kit comprising:
a) one or more oligonucleotide primer groups, each group comprising one or more oligonucleotide primer sets, each set **characterised by**:
i) a first oligonucleotide primer (forward primer) comprising a 5' upstream target-specific or self-annealing portion, a fluorescence cassette-specific portion and a 3' downstream target-specific portion; and
ii) one or more target-specific oligonucleotide primers;
wherein the oligonucleotide primers in a particular set are each suitable for hybridisation to a strand of a target polynucleotide to permit formation of an amplification product of the target polynucleotide in an isothermal polynucleotide amplification reaction;
and wherein the first oligonucleotide primer of each set in the same group contains a fluorescence cassette-specific portion that is capable of hybridising to the complement of the fluorescence cassette-specific portion of the first oligonucleotide primer of any set in the same group;
b) one or more cassette oligonucleotide sets, each set **characterised by**:
i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
ii) a second cassette oligonucleotide labelled with an acceptor moiety (for example a quencher moiety);
wherein the cassette oligonucleotides of each set hybridise to one another to form a fluorescent quenched pair.

13. A kit suitable for use in a method for the detection of one or more polynucleotide amplification products, the kit comprising:
a) one or more oligonucleotide primer groups, each group comprising one or more oligonucleotide primer sets, each set **characterised by**:
i) a first oligonucleotide primer (forward primer) comprising a fluorescence cassette-specific portion and a 3' downstream target-specific portion; and
ii) one or more target-specific oligonucleotide primers;
wherein the oligonucleotide primers in a particular set are each suitable for hybridisation to a strand of a target polynucleotide to permit formation of an amplification product of the target polynucleotide in an isothermal polynucleotide amplification reaction;
and wherein the first oligonucleotide primer of each set in the same group contains a fluorescence cassette-specific portion that is capable of hybridising to the complement of the fluorescence cassette-specific portion of the first oligonucleotide primer of any set in the same group;
b) one or more cassette oligonucleotide sets, each set **characterised by**:
i) a first cassette oligonucleotide labelled with a fluorescent moiety (donor moiety) and having a sequence that is capable of hybridisation to the complement of the fluorescence cassette-specific portion of the first oligonucleotide primer of any set in a given oligonucleotide primer group; and
ii) a second cassette oligonucleotide labelled with an acceptor moiety (for example a quencher moiety);
wherein the cassette oligonucleotides of each set hybridise to one another to form a fluorescent quenched pair; and
c) a strand displacement DNA polymerase.

14. The method of any one of claims 1-11 for use in allele specific SNP Genotyping, somatic mutation detection, pathogen detection, gene expression studies or copy number variation studies.

## Patentansprüche

1. Verfahren zum Nachweis von einem oder mehreren Polynukleotid-Amplifikationsprodukten, wobei das Verfahren die folgenden Schritte umfasst:
a) Bereitstellen einer oder mehrerer Oligonukleotid-Primergruppen, wobei jede Gruppe einen oder mehrere Oligonukleotid-Primersätze umfasst, wobei jeder Satz **gekennzeichnet ist durch**:
i) einen ersten Oligonukleotid-Primer (Vorwärtsprimer), der einen fluoreszenzkassettenspezifischen Abschnitt und einen 3'-stromabwärts gelegenen, zielspezifischen Abschnitt umfasst; und
ii) einen oder mehrere zielspezifische Oligonukleotid-Primer;
wobei die Oligonukleotid-Primer in einem bestimmten Satz jeweils zur Hybridisierung an einen Strang eines Zielpolynukleotids geeignet sind, um die Bildung eines Amplifikationsprodukts des Zielpolynukleotids in einer isothermalen Polynukleotid-Amplifikationsreaktion zu ermöglichen;
und wobei der erste Oligonukleotid-Primer jedes Satzes in derselben Gruppe einen fluoreszenzkassettenspezifischen Abschnitt enthält, der fähig ist, mit dem Komplement des fluoreszenzkassettenspezifischen Abschnitts des ersten Oligonukleotid-Primers eines beliebigen Satzes in derselben Gruppe zu hybridisieren;
b) Bereitstellen eines oder mehrerer Kassetten-Oligonukleotid-Sätze, wobei jeder Satz **gekennzeichnet ist durch**:
i) ein erstes Kassetten-Oligonukleotid, das mit einem Fluoreszenzanteil (Donoranteil) markiert ist und eine Sequenz aufweist, die zur Hybridisierung mit dem Komplement des fluoreszenzkassettenspezifischen Abschnitts des ersten Oligonukleotid-Primers eines beliebigen Satzes in einer gegebenen Oligonukleotid-Primergruppe fähig ist; und
ii) ein zweites Kassetten-Oligonukleotid, das mit einem Akzeptoranteil (zum Beispiel einem Löscheranteil) markiert ist;
wobei die Kassetten-Oligonukleotide jedes Satzes aneinander hybridisieren, um ein fluoreszenzgelöschtes Paar zu bilden;
c) Initiieren einer isothermalen Polynukleotid-Amplifikationsreaktion in Gegenwart einer Strangverdrängungs-DNA-Polymerase, wodurch (falls das Zielpolynukleotid vorhanden ist) eine komplementäre Sequenz zu mindestens einem Abschnitt des relevanten ersten Oligonukleotid-Primers erzeugt wird, sodass das relevante zweite (Akzeptor-, zum Beispiel Löschermarkierte) Kassetten-Oligonukleotid weniger fähig ist, mit dem relevanten ersten (fluoreszenzmarkierten) Kassetten-Oligonukleotid zu hybridisieren, wodurch ein Signal erzeugt wird; und
d) Nachweis des Signals, das erzeugt wird.

2. Verfahren nach Anspruch 1, wobei der erste Oligonukleotid-Primer (Vorwärtsprimer) einen 5'-stromaufwärts gelegenen, zielspezifischen oder selbsthybridisierenden Abschnitt in Bezug auf den fluoreszenzkassettenspezifischen Abschnitt aufweist.

3. Verfahren nach Anspruch 2, wobei der erste Oligonukleotid-Primer (Vorwärtsprimer) einen 5'-stromaufwärts gelegenen zielspezifischen Abschnitt in Bezug auf den fluoreszenzkassettenspezifischen Abschnitt aufweist, wie etwa wobei die isothermale Polynukleotid-Amplifikationsreaktion eine schleifenvermittelte isothermale Amplifikation (LAMP), eine Kreuz-Priming-Amplifikation (CPA) oder eine Smart-Amplifikation (SMAP) ist.

4. Verfahren nach Anspruch 2, wobei der erste Oligonukleotid-Primer (Vorwärtsprimer) einen 5'-stromaufwärts gelegenen, selbsthybridisierenden Abschnitt in Bezug auf den fluoreszenzkassettenspezifischen Abschnitt aufweist, wie etwa wobei die isothermale Polynukleotid-Amplifikationsreaktion eine Smart-Amplifikation (SMAP) ist.

5. Verfahren nach einem der vorhergehenden Ansprüche, wobei die ersten und zweiten Kassetten-Oligonukleotide in jedem Satz keinen zielspezifischen Abschnitt umfassen.

6. Verfahren nach einem der vorhergehenden Ansprüche, wobei die Tm (Tm A) des fluoreszenzgelöschten Paares oder der fluoreszenzgelöschten Paare innerhalb von (±) 20 °C, wie etwa innerhalb von (±)10 °C der Ta der Amplifikationsreaktion liegt;
wobei optional die Tm (Tm A) des fluoreszenzgelöschten Paares oder der fluoreszenzgelöschten Paare über dem Ta der isothermalen Polynukleotid-Amplifikationsreaktion liegt, wie etwa bis zu 15 °C, bis zu 10 °C, oder zwischen 1 und 10 °C über dem Ta der isothermalen Polynukleotid-Amplifikationsreaktion.

7. Verfahren nach einem der vorhergehenden Ansprüche, wobei eine der ersten oder zweiten Oligonukleotid-Kassetten in jedem Satz eine Tm aufweist, die bei oder unter der Ta der isothermalen Polynukleotid-Amplifikationsreaktion liegt.

8. Verfahren nach einem der vorhergehenden Ansprüche, wobei das Signal am Endpunkt der Reaktion nachgewiesen wird.

9. Verfahren nach einem der Ansprüche 1 bis 7, wobei das Signal in Echtzeit nachgewiesen wird.

10. Verfahren nach einem der vorhergehenden Ansprüche, wobei zumindest eine der Basen von zumindest einem der Oligonukleotide, zum Beispiel zumindest eines der Kassetten-Oligonukleotide, optional das fluoreszenzmarkierte Kassetten-Oligonukleotid, ein Phosphorothioat ist;
wobei optional 20-80 % der Basen zumindest eines der Oligonukleotide, zum Beispiel zumindest eines der Kassetten-Oligonukleotide, optional das fluoreszenzmarkierte Kassetten-Oligonukleotid, Phosphorothioate sind.

11. Verfahren nach einem der vorhergehenden Ansprüche, wobei es zumindest 2 und optional 3, 4, 5, 6, 7, 8, 9 oder 10 Oligonukleotid-Primergruppen und entsprechende Kassetten-Oligonukleotid-Sätze gibt.

12. Kit, das zur Verwendung in einem Verfahren zum Nachweis von einem oder mehreren Polynukleotid-Amplifikationsprodukten geeignet ist, wobei das Kit Folgendes umfasst:
a) eine oder mehrere Oligonukleotid-Primergruppen, wobei jede Gruppe einen oder mehrere Oligonukleotid-Primersätze umfasst, wobei jeder Satz **gekennzeichnet ist durch**:
i) einen ersten Oligonukleotid-Primer (Vorwärtsprimer), der einen 5'-stromaufwärts gelegenen, zielspezifischen oder selbsthybridisierenden Abschnitt, einen fluoreszenzkassettenspezifischen Abschnitt und einen 3'-stromabwärts gelegenen, zielspezifischen Abschnitt umfasst; und
ii) einen oder mehrere zielspezifische Oligonukleotid-Primer;
wobei die Oligonukleotid-Primer in einem bestimmten Satz jeweils zur Hybridisierung an einen Strang eines Zielpolynukleotids geeignet sind, um die Bildung eines Amplifikationsprodukts des Zielpolynukleotids in einer isothermalen Polynukleotid-Amplifikationsreaktion zu ermöglichen;
und wobei der erste Oligonukleotid-Primer jedes Satzes in derselben Gruppe einen fluoreszenzkassettenspezifischen Abschnitt enthält, der fähig ist, mit dem Komplement des fluoreszenzkassettenspezifischen Abschnitts des ersten Oligonukleotid-Primers eines beliebigen Satzes in derselben Gruppe zu hybridisieren;
b) eine oder mehrere Kassetten-Oligonukleotid-Sätze, wobei jeder Satz **gekennzeichnet ist durch**:
i) ein erstes Kassetten-Oligonukleotid, das mit einem Fluoreszenzanteil (Donoranteil) markiert ist und eine Sequenz aufweist, die zur Hybridisierung mit dem Komplement des fluoreszenzkassettenspezifischen Abschnitts des ersten Oligonukleotid-Primers eines beliebigen Satzes in einer gegebenen Oligonukleotid-Primergruppe fähig ist; und
ii) ein zweites Kassetten-Oligonukleotid, das mit einem Akzeptoranteil (zum Beispiel einem Löscheranteil) markiert ist;
wobei die Kassetten-Oligonukleotide jedes Satzes aneinander hybridisieren, um ein fluoreszenzgelöschtes Paar zu bilden.

13. Kit, das zur Verwendung in einem Verfahren zum Nachweis von einem oder mehreren Polynukleotid-Amplifikationsprodukten geeignet ist, wobei das Kit Folgendes umfasst:
a) eine oder mehrere Oligonukleotid-Primergruppen, wobei jede Gruppe einen oder mehrere Oligonukleotid-Primersätze umfasst, wobei jeder Satz **gekennzeichnet ist durch**:
i) einen ersten Oligonukleotid-Primer (Vorwärtsprimer), der einen fluoreszenzkassettenspezifischen Abschnitt und einen 3'-stromabwärts gelegenen, zielspezifischen Abschnitt umfasst; und
ii) einen oder mehrere zielspezifische Oligonukleotid-Primer;
wobei die Oligonukleotid-Primer in einem bestimmten Satz jeweils zur Hybridisierung an einen Strang eines Zielpolynukleotids geeignet sind, um die Bildung eines Amplifikationsprodukts des Zielpolynukleotids in einer isothermalen Polynukleotid-Amplifikationsreaktion zu ermöglichen;
und wobei der erste Oligonukleotid-Primer jedes Satzes in derselben Gruppe einen fluoreszenzkassettenspezifischen Abschnitt enthält, der fähig ist, mit dem Komplement des fluoreszenzkassettenspezifischen Abschnitts des ersten Oligonukleotid-Primers eines beliebigen Satzes in derselben Gruppe zu hybridisieren;
b) eine oder mehrere Kassetten-Oligonukleotid-Sätze, wobei jeder Satz **gekennzeichnet ist durch**:
i) ein erstes Kassetten-Oligonukleotid, das mit einem Fluoreszenzanteil (Donoranteil) markiert ist und eine Sequenz aufweist, die zur Hybridisierung mit dem Komplement des fluoreszenzkassettenspezifischen Abschnitts des ersten Oligonukleotid-Primers eines beliebigen Satzes in einer gegebenen Oligonukleotid-Primergruppe fähig ist; und
ii) ein zweites Kassetten-Oligonukleotid, das mit einem Akzeptoranteil (zum Beispiel einem Löscheranteil) markiert ist;
wobei die Kassetten-Oligonukleotide jedes Satzes aneinander hybridisieren, um ein fluoreszenzgelöschtes Paar zu bilden; und
c) eine Strangverdrängungs-DNA-Polymerase.

14. Verfahren nach einem der Ansprüche 1 bis 11 zur Verwendung bei der allelspezifischen SNP-Genotypisierung, beim somatischen Mutationsnachweis, beim Pathogennachweis, bei Genexpressionsstudien oder bei Kopienzahlvariationsstudien.

## Revendications

1. Procédé de détection d'un ou plusieurs produits d'amplification polynucléotidique, le procédé comprenant les étapes de :
a) fourniture d'un ou plusieurs groupes d'amorces oligonucléotidiques, chaque groupe comprenant un ou plusieurs ensembles d'amorces oligonucléotidiques, chaque ensemble étant **caractérisé par** :
i) une première amorce oligonucléotidique (amorce sens) comprenant une partie spécifique à la cassette de fluorescence et une partie spécifique à la cible en aval en 3' ; et
ii) une ou plusieurs amorces oligonucléotidiques spécifiques à la cible ;
dans lequel les amorces oligonucléotidiques dans un ensemble particulier sont chacune appropriées pour l'hybridation à un brin d'un polynucléotide cible pour permettre la formation d'un produit d'amplification du polynucléotide cible dans une réaction d'amplification polynucléotidique isotherme ;
et dans lequel la première amorce oligonucléotidique de chaque ensemble du même groupe contient une partie spécifique à la cassette de fluorescence qui est capable de s'hybrider au complément de la partie spécifique à la cassette de fluorescence de la première amorce oligonucléotidique de n'importe quel ensemble du même groupe ;
b) la fourniture d'un ou plusieurs ensembles d'oligonucléotides de cassette, chaque ensemble étant **caractérisé par** :
i) un premier oligonucléotide de cassette marqué avec un groupement fluorescent (groupement donneur) et ayant une séquence qui est capable de s'hybrider au complément de la partie spécifique à la cassette de fluorescence de la première amorce oligonucléotidique de n'importe quel ensemble dans un groupe d'amorces oligonucléotidiques donné ; et
ii) un second oligonucléotide de cassette marqué avec un groupement accepteur (par exemple un groupement désactivateur) ;
dans lequel les oligonucléotides de cassette de chaque ensemble s'hybrident les uns aux autres pour former une paire fluorescente désactivée ;
c) l'initiation d'une réaction d'amplification polynucléotidique isotherme en présence d'une ADN polymérase à déplacement de brin générant ainsi (si le polynucléotide cible est présent) une séquence complémentaire d'au moins une partie de la première amorce oligonucléotidique pertinente, de sorte que le second oligonicléotide de cassette pertinent (accepteur, par exemple désactivateur, marqué) est moins capable de s'hybrider au premier oligonucléotide de cassette (marqué par fluorescence) pertinent, moyennant quoi un signal est généré ; et
d) la détection du signal généré.

2. Procédé selon la revendication 1, dans lequel la première amorce oligonucléotidique (amorce sens) a une partie spécifique de la cible ou d'auto-hybridation en amont en 5' par rapport à la partie spécifique à la cassette de fluorescence.

3. Procédé selon la revendication 2, dans lequel la première amorce oligonucléotidique (amorce sens) a une partie spécifique à la cible en amont en 5' par rapport à la partie spécifique à la cassette de fluorescence, de sorte que dans lequel la réaction d'amplification polynucléotidique isotherme est une amplification isotherme médiée par boucle (LAMP), une amplification à amorçage croisé (CPA) ou l'amplification intelligente (SMAP).

4. Procédé selon la revendication 2, dans lequel la première amorce oligonucléotidique (amorce sens) a une partie d'auto-hybridation en amont en 5' par rapport à la partie spécifique à la cassette de fluorescence, de sorte que dans lequel la réaction d'amplification polynucléotidique isotherme est une amplification intelligente (SMAP).

5. Procédé selon l'une quelconque des revendications précédentes, dans lequel les premier et second oligonucléotides de cassette dans chaque ensemble ne comprennent pas de partie spécifique à la cible.

6. Procédé selon l'une quelconque des revendications précédentes, dans lequel la Tm (Tm A) de la paire ou des paires fluorescentes désactivées est à (±) 20 °C, comme à (±) 10 °C de la Ta de la réaction d'amplification ; éventuellement
dans lequel la Tm (Tm A) de la paire ou des paires fluorescentes désactivées est supérieure à la Ta de la réaction d'amplification polynucléotidique isotherme, comme jusqu'à 15 °C, jusqu'à 10 °C, ou entre 1 et 10 °C au-dessus de la Ta de la réaction d'amplification polynucléotidique isotherme.

7. Procédé selon l'une quelconque des revendications précédentes, dans lequel l'une parmi les première ou seconde cassettes d'oligonucléotides dans chaque ensemble a une Tm qui est égale ou inférieure à la Ta de la réaction d'amplification polynucléotidique isotherme.

8. Procédé selon l'une quelconque des revendications précédentes, dans lequel le signal est détecté au point final de la réaction.

9. Procédé selon l'une quelconque des revendications 1 à 7, dans lequel le signal est détecté en temps réel.

10. Procédé selon l'une quelconque des revendications précédentes, dans lequel au moins une des bases d'au moins un des oligonucléotides, par exemple au moins un des oligonucléotides de cassette, éventuellement l'oligonucléotide de cassette marqué par fluorescence, est un phosphorothioate ; éventuellement
dans lequel 20 à 80 % des bases d'au moins un des oligonucléotides, par exemple au moins un des oligonucléotides de cassette, éventuellement l'oligonucléotide de cassette marqué par fluorescence, sont des phosphorothioates.

11. Procédé selon l'une quelconque des revendications précédentes, dans lequel il existe au moins 2 et éventuellement 3, 4, 5, 6, 7, 8, 9 ou 10 groupes d'amorces oligonucléotidiques et ensembles d'oligonucléotides de cassette correspondants.

12. Kit convenant pour une utilisation dans un procédé de détection d'un ou plusieurs produits d'amplification polynucléotidique, le kit comprenant :
a) un ou plusieurs groupes d'amorces oligonucléotidiques, chaque groupe comprenant un ou plusieurs ensembles d'amorces oligonucléotidiques, chaque ensemble étant **caractérisé par** :
i) une première amorce oligonucléotidique (amorce sens) comprenant une partie spécifique à la cible ou auto-hybridation en amont en 5', une partie spécifique à la cassette de fluorescence et une partie spécifique à la cibleen aval en 3' ; et
ii) une ou plusieurs amorces oligonucléotidiques spécifiques à la cible ;
dans lequel les amorces oligonucléotidiques dans un ensemble particulier sont chacune appropriées pour l'hybridation à un brin d'un polynucléotide cible pour permettre la formation d'un produit d'amplification du polynucléotide cible dans une réaction d'amplification polynucléotidique isotherme ;
et dans lequel la première amorce oligonucléotidique de chaque ensemble du même groupe contient une partie spécifique à la cassette de fluorescence qui est capable de s'hybrider au complément de la partie spécifique à la cassette de fluorescence de la première amorce oligonucléotidique de n'importe quel ensemble du même groupe ;
b) un ou plusieurs ensembles d'oligonucléotides de cassette, chaque ensemble étant **caractérisé par** :
i) un premier oligonucléotide de cassette marqué avec un groupement fluorescent (groupement donneur) et ayant une séquence qui est capable de s'hybrider au complément de la partie spécifique à la cassette de fluorescence de la première amorce oligonucléotidique de n'importe quel ensemble dans un groupe d'amorces oligonucléotidiques donné ; et
ii) un second oligonucléotide de cassette marqué avec un groupement accepteur (par exemple un groupement désactivateur) ;
dans lequel les oligonucléotides de cassette de chaque ensemble s'hybrident les uns aux autres pour former une paire fluorescente désactivée.

13. Kit convenant pour une utilisation dans un procédé de détection d'un ou plusieurs produits d'amplification polynucléotidique, le kit comprenant :
a) un ou plusieurs groupes d'amorces oligonucléotidiques, chaque groupe comprenant un ou plusieurs ensembles d'amorces oligonucléotidiques, chaque ensemble étant **caractérisé par** :
i) une première amorce oligonucléotidique (amorce sens) comprenant une partie spécifique à la cassette de fluorescence et une partie spécifique à la cible en aval en 3' ; et
ii) une ou plusieurs amorces oligonucléotidiques spécifiques à la cible ;
dans lequel les amorces oligonucléotidiques dans un ensemble particulier sont chacune appropriées pour l'hybridation à un brin d'un polynucléotide cible pour permettre la formation d'un produit d'amplification du polynucléotide cible dans une réaction d'amplification polynucléotidique isotherme ;
et dans lequel la première amorce oligonucléotidique de chaque ensemble du même groupe contient une partie spécifique à la cassette de fluorescence qui est capable de s'hybrider au complément de la partie spécifique à la cassette de fluorescence de la première amorce oligonucléotidique de n'importe quel ensemble du même groupe ;
b) un ou plusieurs ensembles d'oligonucléotides de cassette, chaque ensemble étant **caractérisé par** :
i) un premier oligonucléotide de cassette marqué avec un groupement fluorescent (groupement donneur) et ayant une séquence qui est capable de s'hybrider au complément de la partie spécifique à la cassette de fluorescence de la première amorce oligonucléotidique de n'importe quel ensemble dans un groupe d'amorces oligonucléotidiques donné ; et
ii) un second oligonucléotide de cassette marqué avec un groupement accepteur (par exemple un groupement désactivateur) ;
dans lequel les oligonucléotides de cassette de chaque ensemble s'hybrident les uns aux autres pour former une paire fluorescente désactivée ; et
c) une ADN polymérase à déplacement de brin.

14. Procédé selon l'une quelconque des revendications 1 à 11 destiné à être utilisé dans le génotypage SNP spécifique d'un allèle, la détection de mutations somatiques, la détection d'agents pathogènes, les études d'expression génique ou les études de variation du nombre de copies.
